**Europäisches Patentamt**

**European Patent Office**

**Office européen des brevets**

(19)

(11) Veröffentlichungsnummer : **0 083 593
B1**

(12) **EUROPÄISCHE PATENTSCHRIFT**

(45) Veröffentlichungstag der Patentschrift :
**26.09.84**

(21) Anmeldenummer : **82901090.9**

(22) Anmeldetag : **19.04.82**

(86) Internationale Anmeldenummer :
**PCT/AT 82/00011**

(87) Internationale Veröffentlichungsnummer :
**WO/8300367 (03.02.83 Gazette 83/04)**

(51) Int. Cl.³ : **F 16 K   1/14**, F 16 K  15/04,
**A 61 J   1/00**, A 61 M  25/00

(54) **VENTIL ZUR ZUFÜHRUNG, ÜBERPRÜFUNG UND ENTNAHME EINES IN EINEM IMPLANTIERTEN BEHÄLTER ENTHALTENEN MEDIUMS.**

(30) Priorität : **16.07.81 AT 3137/81**

(43) Veröffentlichungstag der Anmeldung :
**20.07.83 Patentblatt 83/29**

(45) Bekanntmachung des Hinweises auf die Patenterteilung : **26.09.84 Patentblatt 84/39**

(84) Benannte Vertragsstaaten :
**BE CH DE FR GB LI NL SE**

(56) Entgegenhaltungen :
**DE-A- 1 650 538
FR-A- 1 327 106**

(73) Patentinhaber : **TRAWÖGER, Werner
Höttinger Au Nr. 60
A-6020 Innsbruck (AT)**

(72) Erfinder : **TRAWÖGER, Werner
Höttinger Au Nr. 60
A-6020 Innsbruck (AT)**

(74) Vertreter : **Torggler, Paul, Dr. et al
Patentanwälte Dr. Paul Torggler DDr. Engelbert
Hofinger Wilhelm-Greil-Strasse 16
A-6020 Innsbruck (AT)**

## Beschreibung

Die Erfindung bezieht sich auf ein Ventil zur Zuführung, Überprüfung und Entnahme eines in einem implantierten Behälter enthaltenen Mediums, mit einem Ventilkörper, in dem ein Durchflußkanal vorgesehen ist, der sich zur Einführung einer Injektionsnadel nach außen konisch erweitert und in einen mit dem Behälter in Verbindung stehenden Raum mündet, und in den ein mit einem Rückstellmittel beaufschlagtes Verschlußglied eingesetzt ist, das durch die Einführung der Injektionsnadel den Durchflußkanal freigibt und beim Herausziehen wieder verschließt.

## Darlegung des bekannten Standes der Technik

Ein derartiges Ventil ist beispielsweise aus der AT-A-345.438 bekannt geworden, die einen implantierbaren Behälter zur langandauernden Abgabe von Medikamenten od. dgl. beschreibt. Das Ventil ist ein Federventil und als Bestandteil des Behälters in eine sich nach außen konisch erweiternde Bohrung eingesetzt. Zum Auffüllen des implantierten Behälters wird das Ventil beim Einstich mit einer Injektionsnadel geöffnet und verschließt sich beim Herausziehen wieder selbsttätig. Da die gerade, das Ventilgehäuse durchsetzende Bohrung nicht nur das Verschlußglied sondern auch die Rückstellfeder aufnehmen muß und beide Teile beim Einstechen der Injektionsnadel in der Bohrung verbleiben müssen, hat sich gezeigt, daß die Funktion dieses Ventils auf die Dauer nicht den Anforderungen entspricht.

Die Aufgabe der Erfindung liegt daher darin, ein einfach herzustellendes Ventil dieser Art mit wesentlich verlängerter Lebensdauer zu schaffen, bei dem das Einführen der Injektionsnadel nicht durch Ventilteile behindert wird, sodaß keine Störungen auch bei oftmaliger Betätigung auftreten, da Reparaturen bzw. ein Austausch des Implanates so weit als möglich ausgeschlossen sein sollen, um den Patienten nicht unnötig zu belasten. Das auf diese Weise zu schaffende Ventil soll weiters nicht nur fester Bestandteil eines implantierten Behälters sein, sondern in einer anderen Ausführung auch als eigener Bauteil in den Körper einsetzbar sein und über eine Schlauchleitung mit dem implantierten Behälter verbindbar sein.

## Darlegung des Wesens der Erfindung

Erfindungsgemäß wird diese Aufgabe dadurch gelöst, daß der Durchflußkanal sich aus einem als erste Sackbohrung ausgebildeten Einführungskanal für die Injektionsnadel und einem im Winkel dazu in den Einführungskanal mündenden zweiten Sackbohrung zusammengesetzt, die das Verschlußglied aufnimmt, wobei das Verschlußglied im Schließzustand in den Einführungskanal ragt.

Beim erfindungsgemäßen Ventil verläuft der Durchflußkanal über Eck, wobei das Verschlußglied durch die eingeführte Injektionsnadel seitlich weggedrückt und damit vom Ventilsitz abgehoben wird. Das Rückstellmittel ist daher nicht in den Einführungskanal eingesetzt und behindert nicht die Einführung der Injektionsnadel, die beim Einbau einer Feder in den Einführungskanal auf Dauer unvermeidlich sind. Auch eine oftmalige Betätigung des Ventils führt zu keinen durch das Einsetzen der Injektionsnadel bedingten Störungen.

In einer ersten Ausführung ist vorgesehen, daß das Verschlußglied ein rotationssymmetrischer Körper ist, dessen Durchmesser geringer als der der zweiten Sackbohrung ist und der sich zum Einführungskanal hin verjüngt, wobei als Ventilsitz ein O-Ring vorgesehen ist, der aus einer Ringnut der zweiten Sackbohrung radial nach innen vorsteht.

Das Verschlußglied, das insbesondere als Kugel ausgebildet ist, steht in einer weiteren Ausführung bevorzugt aus der zweiten Sackbohrung in den mit dem Behälter in Verbindung stehenden Raum vor, wobei der vorstehende Teil durch ein elastisches Element beaufschlagt ist. Das elastische Element ist vorzugsweise als Ring ausgebildet und der Ventilkörper zumindest im Bereich der zweiten Sackbohrung zylindrisch ausgebildet, wobei der Ring in diesem zylindrischen Bereich den Ventilkörper umschließt und auf den vorstehenden Teil des Verschlußgliedes drückt.

## Beschreibung der Zeichnungsfiguren

Nachstehend wird nun die Erfindung an Hand der Figuren der beiliegenden Zeichnungen in einem bevorzugten Ausführungsbeispiel näher beschrieben, ohne darauf beschränkt zu sein. Es zeigen :

Figur 1 einen Schnitt durch ein als eigener Bauteil implantiertes Ventil in der Schließstellung, und

Figur 2 dasselbe in Offenstellung mit eingesetzter Injektionsnadel.

## Beschreibung eines bevorzugten Ausführungsbeispieles

Das erfindungsgemäße Ventil weist einen Ventilkörper 2 auf, der im gezeigten Ausführungsbeispiel in ein implantiertes Ventilgehäuse 1 eingesetzt ist. Das Ventilgehäuse 1 könnte auch durch einen implantierten Vorratsbehälter gebildet sein, sodaß der Ventilkörper 2 direkt im Behälter angeordnet ist. Der Ventilkörper 2 ist im wesentlichen zylindrisch ausgebildet und weist eine ringförmige Grundplatte größeren Durchmessers auf, die mittels Schrauben 13 mit dem Ventilgehäuse 1 oder dem Behälter verbindbar ist.

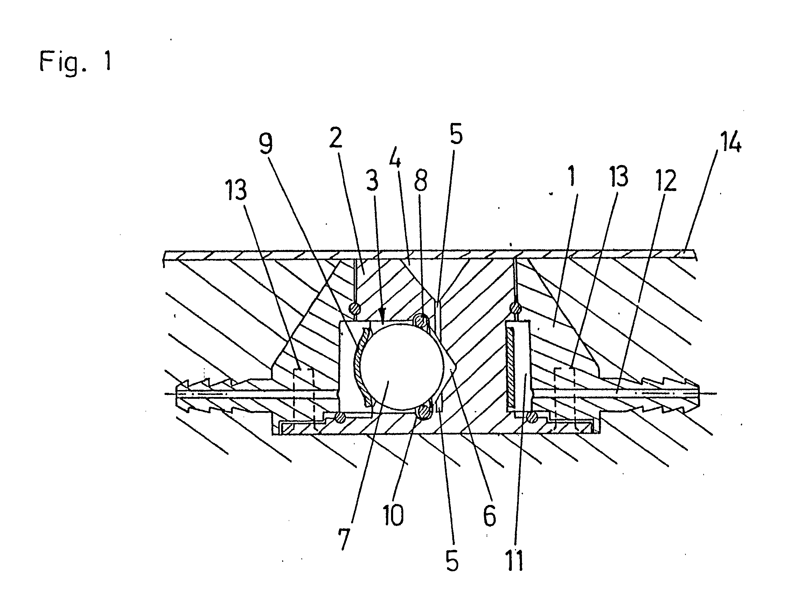

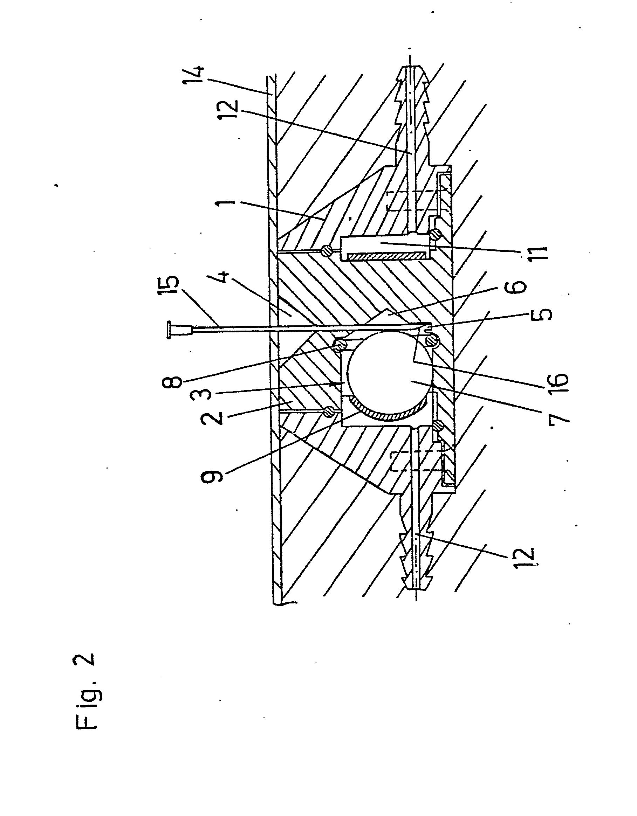

Das Ventilgehäuse 1 ist mit einer zylindrischen Bohrung versehen, die sich zu einem ringförmigen Raum 11 erweitert, von dem aus zumindest eine radiale Bohrung 12 durch einen angeformten Leitungsanschluß nach außen führt, an den ein Verbindungsschlauch zum implantierten Behälter anschließbar ist. Im dargestellten Ausführungsbeispiel sind zwei derartige Leitungsanschlüsse vorgesehen. Ein derartiges Ventil kann daher in eine im Körper verlegte Leitung eingesetzt werden, die beispielsweise zwischen einem von außen komprimierbaren Vorratsbehälter und einer Druckmanschette verläuft, die ein röhrenförmiges Körperorgan zu dessen Querschnittsveränderung umgibt. Der Ventilkörper 2 weist eine axial verlaufende Sackbohrung auf, die einen Einführungskanal 5 für eine einzusetzende Injektionsnadel 15 (Fig. 2) darstellt und eine sich zur Haut 14 hin konische Erweiterung 4 hinweist, um beim Durchstechen der Haut 14 die Nadel leichter in den Einführungskanal 5 einführen zu können. Eine zweite Sackbohrung 6 mündet radial in den Einführungskanal 5 und nimmt ein als Kugel ausgebildetes Verschlußglied 7 auf. Der Durchmesser des Verschlußgliedes 7 ist geringfügig kleiner als der Durchmesser der Sackbohrung 6, sodaß ein Durchflußkanal 3 freibleibt. Als Ventilsitz 8 dient ein O-Ring, der in eine Ringnut 10 der Sackbohrung 6 eingesetzt ist und nach innen vorsteht. Das Verschlußglied 7 ragt mit seinem innersten Bereich in den Einführungkanal 5, während es im äußersten Bereich aus der Sackbohrung 6 in den Raum 11 vorsteht. Als Rückstellmittel dient ein elastischer Ring 9, der den zylindrischen Ventilkörper 2 im Bereich der Sackbohrung 6 umgibt und am vorstehenden Bereich des Verschlußgliedes 7 anliegt. Zur Fixierung des Ringes 9 am Ventilkörper 2 ist dieser in diesem Bereich mit einer Ringnut versehen. Im Schließzustand nach Fig. 1 ist das Verschlußglied 7 durch den Ring 9 an den Ventilsitz 8 angedrückt. Wird nun entsprechend Fig. 2 eine Injektionsnadel 15 in den Einführungskanal 5 eingesetzt, so wird der in den Einführungskanal 5 ragende Teil des Verschlußgliedes 7 verdrängt und dieses vom Ventilsitz 8 abgehoben, wobei es durch die Bewegung der Injektionsnadel 15 an den unteren Wandungsbereich der Sackbohrung 6 angedrückt wird. Die zuzuführende Flüssigkeit tritt nun aus dem Auslaß 16 der Injektionsnadel 15 aus und tritt durch den freigegebenen Durchflußkanal 3 in den Raum 11 ein, aus dem es über die Bohrungen 12 in eine angesetzte Schlauchleitung gließen kann. Nach dem Herausziehen der Injektionsnadel 15 drückt der elastische Ring 9 das Verschlußglied 7 weiter in den Ventilsitz 8, womit das Ventil verschlossen ist.

Anstelle der Nachfüllung von Flüssigkeit kann diese selbstverständlich auch in gleicher Weise entnommen werden bzw. deren Druck überprüft werden, wenn an die Injektionsnadel 15 ein Druckmeßgerät angeschlossen wird. Wird das Ventil als eigener Bauteil in einem gesonderten Ventilgehäuse 1 unmittelbar unter die Haut 14 implantiert, so ist dessen Oberfläche vorzugsweise mit leicht fühlbaren Griffmulden versehen, sodaß die Injektionsnadel 15 in den die Erweiterung 4 unmittelbar unter der Haut 14 aufweisenden Einführungkanal 5 ohne Schwierigkeiten eingestochen werden kann.

Die der Haut 14 zugewandte Oberfläche des Ventilkörpers 2, in die die Erweiterung 4 eingelassen ist, kann gegebenenfalls noch mit einer nicht gezeigten Beschichtung, beispielsweise einer dünnen Silikonhaut, versehen sein, die auch die Erweiterung 4 überdeckt und von der Injektionsnadel durchstoßen wird. Durch diese Beschichtung kann ein eventuell denkbares Zuwachsen der Erweiterung und gegebenenfalls auch der Öffnung des Einführungskanales 5 durch die Haut 14 vermieden werden.

## Ansprüche

1. Ventil zur Zuführung, Überprüfung und Entnahme eines in einem implantierten Behälter enthaltenen Mediums, mit einem Ventilkörper (2), in dem ein Durchflußkanal (3) vorgesehen ist, der sich zur Einführung einer Injektionsnadel (15) nach außen konisch erweitert und in einen mit dem Behälter in Verbindung stehenden Raum (11) mündet, und in den ein mit einem Rückstellmittel (9) beaufschlagtes Verschlußglied (7) eingesetzt ist, das durch die Einführung der Injektionsnadel (15) den Durchflußkanal (3) freigibt und beim Herausziehen wieder verschließt, dadurch gekennzeichnet, daß der Durchflußkanal (3) sich aus einem als erste Sackbohrung ausgebildeten Einführungskanal (5) für die Injektionsnadel (15) und einen im Winkel dazu in den Einführungskanal (5) mündenden zweiten Sackbohrung (6) zusammengesetzt, die das Verschlußglied (7) aufnimmt, wobei das Verschlußglied (7) im Schließzustand in den Einführungskanal (5) ragt.

2. Ventil nach Anspruch 1, dadurch gekennzeichnet, daß die zweite Sackbohrung (6) sich radial vom Einführungskanal (5) erstreckt.

3. Ventil nach Anspruch 1 oder 2, dadurch gekennzeichnet, daß das Verschlußglied (7) ein rotationssymmetrischer Körper ist, dessen Durchmesser geringer als der der zweiten Sackbohrung (6) ist und der sich zum Einführungskanal (5) hin verjüngt, wobei als Ventilsitz (8) ein O-Ring vorgesehen ist, der aus einer Ringnut (10) der zweiten Sackbohrung (6) radial nach innen vorsteht.

4. Ventil nach einem der Ansprüche 1 bis 3, dadurch gekennzeichnet, daß das Verschlußglied (7) aus der zweiten Sackbohrung (6) in den mit dem Behälter in Verbindung stehenden Raum (11) vorsteht und der vorstehende Teil von einem elastischen Element beaufschlagt ist.

5. Ventil nach einem der Ansprüche 1 bis 4, dadurch gekennzeichnet, daß der Ventilkörper (2) zumindest im Bereich der zweiten Sackbohrung (6) zylindrisch und von einem elastischen Ring (9) umgeben ist, der den vorstehenden Teil des Verschlußgliedes (7) beaufschlagt.

6. Ventil nach einem der Ansprüche 1 bis 5, dadurch gekennzeichnet, daß das Verschlußglied (7) eine Kugel ist.

7. Ventil nach einem der Ansprüche 1 bis 6, dadurch gekennzeichnet, daß es in einem unter die Haut implantierbaren Ventilgehäuse (1) angeordnet ist, das einen Leitungsanschluß (12) für die Verbindung zum implantierten Behälter aufweist.

## Claims

1. Valve for supplying, controlling and taking out a medium contained in an implanted container, comprising a valve body (2), wherein a flow channel (3) is provided which widens conically towards the outer side for the introduction of an injection needle (15) and runs into a chamber (11) connected with the container, and in which a closure member (7) acted upon by a reset means (9) is inserted which clears the flow channel (3) when the injection needle (15) is inserted and recloses when it is taken out, characterized in that the flow channel (3) comprises an introduction channel (5) for the injection needle (15) in the form of a first pocket bore and a second bocket bore (6) angularly arranged thereto and leading into the introduction channel (5), which second pocket bore receives the closure member (7), whereby the closure member (7) projects into the introduction channel (5) in the closed condition.

2. Valve according to claim 1, characterized in that the second pocket bore (6) extends radially from the introduction channel (5).

3. Valve according to claim 1 or 2, characterized in that the closure member (7) is a solid of revolution whose diameter is smaller than the one of the second pocket bore (6) and which tapers towards the introduction channel (5), whereby one O-ring is provided as a valve seat (8) which projects radially inwardly from an annular groove (10) of the second pocket bore (6).

4. Valve according to claims 1 to 3, characterized in that the closure member (7) projects from the second pocket bore (6) into the chamber (11) connected with the container and the projecting part is acted upon by an elastic element.

5. Valve according to one of claims 1 to 4, characterized in that the valve body (2) is cylindrical at least in the region of the second pocket bore (6) and surrounded by an elastic ring (9) which acts upon the projecting part of the closure member (7).

6. Valve according to one of claims 1 to 5, characterized in that the closure member (7) is a ball.

7. Valve according to one of claims 1 to 6, characterized in that it is arranged in a valve housing (1) implantable under the skin which has a pipe connector (12) for being connected with the implanted container.

## Revendications

1. Soupape pour l'introduction, le contrôle et le prélèvement d'une substance enfermée dans un récipient implanté, pourvu d'un corps de soupape (2) dans lequel est pratiquée une canalisation de passage (3) qui s'évase vers l'extérieur pour l'introduction d'une aiguille d'injection et débouche dans une chambre (11) en communication avec le récipient, et dans laquelle est introduit un élément de fermeture (7) muni d'un élément de rappel (9), de manière à ouvrir la canalisation de passage (3) à la suite de l'introduction de l'aiguille d'injection (15) et rétablir la fermeture à la suite du retrait de celle-ci, caractérisée en ce que la canalisation de passage (3) se compose d'un canal d'entrée (5) pour l'aiguille d'injection (15) en forme de premier alésage borgne et d'un second alésage borgne (6) formant un angle à son intersection avec le canal d'entrée (5), et recevant l'élément de fermeture (7) de sorte que en position de fermeture, cet élément de fermeture (7) s'élève dans le canal d'entrée (5).

2. Soupape selon la revendication 1, caractérisée en ce que le second alésage borgne (6) s'étend radialement par rapport au canal d'entrée (5).

3. Soupape selon l'une des revendications 1 et 2, caractérisée en ce que l'élément de fermeture (7) est une pièce symétrique en rotation dont le diamètre est inférieur à celui du second alésage borgne (6) qui se rétrécit en direction du canal d'entrée (5), tandis qu'un anneau torique formant siège de soupape (8) fait saillie, en direction radiale, d'une gorge circulaire (10) formée dans le second alésage borgne (6).

4. Soupape selon l'une des revendications 1 à 3, caractérisée en ce que l'élément de fermeture (7) fait saillie depuis le second alésage borgne (6) jusque dans la chambre (11) en communication avec le réservoir et en ce que la partie en saillie est sollicitée par un élément élastique.

5. Soupape selon l'une des revendications 1 à 4, caractérisée en ce que le corps de soupape (2) est cylindrique au moins dans la zone du second alésage borgne (6) et est entouré d'un bracelet élastique (9) qui est appliqué contre la partie en saillie de l'élément de fermeture (7).

6. Soupape selon l'une des revendications 1 à 5, caractérisée en ce que l'élément de fermeture (7) est une bille.

7. Soupape selon l'une des revendications 1 à 6, caractérisée en ce qu'elle est disposée dans un corps de soupape (1) implanté sous la peau, et qui présente un raccord de canalisation (12) pour la communication avec le réservoir implanté.

Fig. 1

Fig. 2

0 083 593